(19) Europäisches Patentamt
European Patent Office
Office européen des brevets

(11) Veröffentlichungsnummer: **0 049 688**
**B1**

(12) **EUROPÄISCHE PATENTSCHRIFT**

(45) Veröffentlichungstag der Patentschrift: **16.01.85**

(51) Int. Cl.⁴: **C 07 D 207/263,** C 07 D 211/76, C 07 D 223/10

(21) Anmeldenummer: **81810397.0**

(22) Anmeldetag: **01.10.81**

# (54) Verfahren zur Herstellung von N-Cyanlactamen.

(30) Priorität: **07.10.80 CH 7480/80**

(43) Veröffentlichungstag der Anmeldung: **14.04.82 Patentblatt 82/15**

(45) Bekanntmachung des Hinweises auf die Patenterteilung: **16.01.85 Patentblatt 85/3**

(84) Benannte Vertragsstaaten: **DE FR GB NL**

(56) Entgegenhaltungen:
**DE - A - 1 670 851**
**DE - A - 1 905 098**
**DE - B - 1 175 678**

**HOUBEN-WEYL "Methoden der organischen Chemie", 4. Auflage Band VIII, 1952 GEORG THIEME VERLAG, Stuttgart, Seite 177**
**Römpps Chemie (1975), S. 3114; Houben-Weyl, Band XI/2, (1958), S. 518-526**

(73) Patentinhaber: **CIBA-GEIGY AG, Postfach, CH-4002 Basel (CH)**

(72) Erfinder: **Trachsler, Dieter, Dr., Liebrütistrasse 21/15, CH-4303 Kaiseraugst (CH)**
Erfinder: **Lohse, Friedrich, Prof. Dr., Buchenstrasse 23, CH-4104 Oberwil (CH)**

**EP 0 049 688 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein neues Verfahren zur Herstellung von N-Cyanlactamen.

Für die Herstellung von N-Cyanlactamen sind bereits mehrere Verfahren bekannt.

So werden beispielsweise gemäss DE-OS 1 670 851 Lactim-O-alkyläther mit Derivaten des Kohlensäuremonochlorids in Abwesenheit eines Lösungsmittels oder in Gegenwart eines inerten wasserfreien organischen Lösungsmittels umgesetzt, und dabei Lactam-N-carbonsäurederivate, erhalten. N-Cyanlactam ist erwähnt, aber nicht durch Beispiele belegt. Hingegen enthält DE-OS 1 905 098, welche ein Verfahren zur Herstellung von Polyamiden in Gegenwart von N-substituierten Lactamen als Aktivatoren offenbart, ein Beispiel für die Herstellung von N-Cyanocaprolactam aus Caprolactim-O-methyläther und Chlorcyan in Gegenwart eines Lösungsmittels.

Nach dem in der DE-PS 1 175 678 beschriebenen Verfahren werden N-Cyanlactame dadurch erhalten, dass man Salze von Lactamen in Gegenwart von organischen Lösungsmitteln mit Halogencyanen umsetzt.

Die bisher bekannten Verfahren liefern nur mässige Ausbeuten und benötigen zum Teil den Einsatz von inerten organischen Lösungsmitteln.

Es wurde nun gefunden, dass man N-Cyanlactame in hohen Ausbeuten erhält, wenn man ω-Halogenacylhalogenide mit Cyanamiden bzw. deren Salzen zu einer Ringschlussreaktion bringt.

Gegenstand der vorliegenden Erfindung ist somit ein Verfahren zur Herstellung von N-Cyanlactamen der Formel I

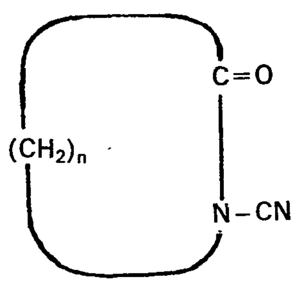

(I),

worin n eine ganze Zahl von 1 bis 5 bedeutet und die Methylenkohlenstoffatome gegebenenfalls durch eine oder zwei Methyl- oder Äthylgruppen substituiert sind oder zwei benachbarte Methylenkohlenstoffatome Bestandteil eines $C_5$–$C_8$ cycloaliphatischen Ringes oder Benzolringes sind, dadurch gekennzeichnet, dass man eine Verbindung der Formel II

$$X{-}\overset{\overset{\displaystyle O}{\|}}{C}{-}(CH_2)_n{-}X \qquad (II),$$

worin n eine ganze Zahl von 3 bis 5 ist, die Methylenkohlenstoffatome gegebenenfalls durch eine oder zwei Methyl- oder Äthylgruppen substituiert sind oder zwei benachbarte Methylenkohlenstoffatome Bestandteil eines $C_5$–$C_8$ cycloaliphatischen Ringes oder Benzolringes sind, und die Symbole X unabhängig voneinander für ein Halogenatom stehen, in Gegenwart eines anorganischen Halogenwasserstoffakzeptors mit Cyanamid oder einem Cyanamidsalz umsetzt.

Bevorzugt verfährt man so, dass man 1 Mol der Verbindung der Formel II mit 1 Mol Cyanamid in Gegenwart von 2 Mol eines anorganischen Halogenwasserstoffakzeptors oder 1 Mol eines Cyanamidsalzes in Gegenwart von 1 Mol eines anorganischen Halogenwasserstoffakzeptors umsetzt.

Die Symbole X in der Formel II können unabhängig voneinander für Chlor, Brom oder Jod stehen, insbesondere für Chlor oder Brom.

Als $C_5$–$C_8$ cycloaliphatische Ringe, in welchen zwei benachbarte Methylenkohlenstoffatome Bestandteil sind, kommen z.B. Cyclopentyl, Cyclohexyl oder Cyclooctyl in Frage.

Beispiele für Verbindungen der Formel II sind: γ-Chlorbuttersäurechlorid, γ-Brombuttersäurechlorid, δ-Chlorvaleriansäurechlorid, δ-Bromvaleriansäurechloride, ε-Chlorhexansäurechlorid, ε-Bromhexansäurechlorid, γ-Chlorvaleriansäurechlorid, 2-Chlormethylbenzoylchlorid sowie die entsprechenden Bromide.

Bevorzugt verwendet man Verbindungen der Formel II, welche unsubstituiert sind und keine Ringe aufweisen.

Das erfindungsgemässe Verfahren dient vorzugsweise zur Herstellung von N-Cyanpyrrolidon, N-Cyanpiperidon und N-Cyancaprolactam.

Als Reaktionspartner der genannten ω-Halogenacylhalogenide der Formel II dienen Cyanamid oder Salze des Cyanamids. Das Cyanamid kann in fester Form oder als wässrige Lösung eingesetzt werden. Als Cyanamidsalze kommen Salze mit den Alkali- und Erdalkalimetallen in Frage. Bevorzugt sind die Salze mit den Alkalimetallen, insbesondere mit Natrium und Kalium. Die Cyanamidsalze entstehen vorzugsweise während der Reaktion, wenn das Cyanamid mit der als Halogenwasserstoffakzeptor wirkenden Base in Berührung kommt.

Geeignete Basen als Halogenwasserstoffakzeptoren sind zum Beispiel die Hydroxide, Carbonate und Bicarbonate der Alkali- und Erdalkalimetalle. Bevorzugt werden Alkalimetallhydroxide, insbesondere Natriumhydroxid oder Kaliumhydroxid als Halogenwasserstoffakzeptor verwendet.

Das Verfahren wird vorzugsweise so durchgeführt, dass man etwa äquivalente Mengen der Reaktanten bei 0–10 °C zusammenführt und das Reaktionsgemisch anschliessend auf 30–100 °C erwärmt.

Die Reaktion kann in einer wässrigen oder organischen Lösung erfolgen. Als organische Lösungsmittel kommen vorzugsweise aprotische Lösungsmittel wie Dimethylformamid und Dimethylacetamid in Frage. Arbeitet man in organischer Lösung, so wird das Cyanamid vorzugsweise in Form eines Salzes, wie z.B. Mono- oder Dinatriumcyanamid eingesetzt.

Bevorzugt ist ein Verfahren, worin die Umsetzung in einer wässrigen Lösung erfolgt.

Das N-Cyanlactam der Formel I kann durch Eindampfen der Lösung und Extraktion des Rückstandes mit einem organischen Lösungsmittel isoliert werden. Als Lösungsmittel eignen sich mit Wasser nicht mischbare Lösungsmittel, so z.B.: aromatische Kohlenwasserstoffe, wie z.B. Toluol oder Xylol; halogenierte aromatische Kohlenwasserstoffe, wie z.B. Chlorbenzol, Dichlorbenzol, Chlornaphthalin; Äther wie Diäthyl-, Diisopropyl- und Di-n-butyläther, Dioxan oder Diphenyläther; Ester wie n-Butylacetat.

Arbeitet man in wässriger Lösung, so kann man bereits nach Bildung des Cyanamidsalzes das Lösungsmittel zugeben und die Extraktion gleichzeitig mit der Ringschlussreaktion durchführen. Das Rohprodukt wird gegebenenfalls mittels einer geeigneten Reinigungsmethode, wie zum Beispiel Destillation oder Umkristallisation, gereinigt.

Man geht vorzugsweise so vor, dass zu einer auf 0 bis 10 °C gekühlten Natron- oder Kalilauge (4 N oder konzentrierter) die entsprechende Menge des Cyanamids in fester Form zudosiert wird, wobei sich in der Lösung das Mononatrium- oder Monokaliumcyanamid bildet. Zu dieser Lösung wird bei 0 °C bis 10 °C das ω-Halogenacylhalogenid der Formel II in äquivalenter Menge bezüglich des eingesetzten Cyanamids zugetropft, wobei auf ausreichende Kühlung geachtet wird, da dieser erste Reaktionsschritt, d.h. die Bildung des intermediär entstehenden Halogenacylcyanamid-Salzes, unter starker Exothermie abläuft. Die Ringschlussreaktion setzt bereits bei 0 °C bis 10 °C ein und wird anschliessend durch Erhöhung der Temperatur vervollständigt, wobei je nach entstandenem Zwischenprodukt schon Temperaturen um 30 bis 40 °C ausreichen, aber auch höhere Temperaturen bis 100 °C angewendet werden können. Der Reaktionsablauf kann durch das folgende Schema illustriert werden:

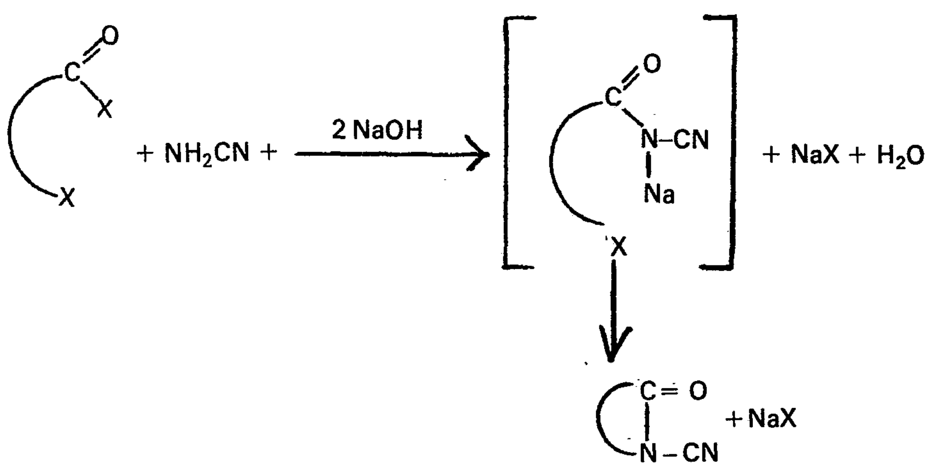


worin X ein Halogenatom bedeutet.

Die ω-Halogenacylhalogenide der Formel II stellen bekannte Verbindungen dar, die grösstenteils im Handel erhältlich sind oder nach bekannten Methoden hergestellt werden können.

Die nach dem erfindungsgemässen Verfahren erhaltenen N-Cyanlactame der Formel I stellen wertvolle Zwischenprodukte dar, z.B. zur Herstellung von vernetzten stickstoffhaltigen Polyaddukten, die durch Addition von organischen Verbindungen mit mindestens 2 C-Atomen und mindestens 2 Hydroxy- und/oder 2 Aminogruppen an N-Cyanlactam entstehen. Sie sind aber auch unmittelbar als Aktivatoren in Polymerisationsreaktionen verwendbar, wie z.B. bei der Herstellung von Polypyrrolidon. Sie können ferner zur Härtung von Epoxidharzen Verwendung finden.

Die folgenden Beispiele erläutern die Erfindung. Prozente sind Gewichtsprozente.

Beispiel 1

80 g (2 Mol) Natriumhydroxid werden in 500 ml Wasser gelöst, und der Lösung werden nach Abkühlen auf 0 °C 42 g (1 Mol) Cyanamid portionenweise zugegeben. Der klaren und farblosen Cyanamidlösung werden 141,0 g (1 Mol) γ-Chlorbuttersäurechlorid während 2 Stunden bei 0 bis 5 °C zugetropft, wobei auf ausreichende Kühlung geachtet wird. Nach beendetem Zutropfen wird während einer weiteren Stunde bei gleicher Temperatur gerührt. Anschliessend wird 1 Liter Methylenchlorid zugesetzt, und das Reaktionsgemisch wird während einer Stunde auf Rückflusstemperatur (40 °C) erwärmt. Die beiden klaren und farblosen Phasen werden im Scheidetrichter getrennt, die organische Phase wird mit Natriumsulfat getrocknet, filtriert, und das Lösungsmittel wird am Wasserstrahlvakuum entfernt.

Ausbeute: 110 g (99,9 % d. Th.). Das Rohprodukt ist eine schwach gelbliche und leicht trübe Flüssigkeit.

Die Hochvakuumdestillation des Rohprodukts ergibt 78,2 g (71 % d.Th.) N-Cyanpyrrolidon, das laut Gaschromatogramm einen Gehalt von 95,85 % an N-Cyanpyrrolidon neben 3,43 % eines Isomeren aufweist, als farblose klare Flüssigkeit vom Siedepunkt 92–94 °C/6,67 Pa.

Beispiel 2

59,4 g (1,484 Mol) Natriumhydroxid werden in 370 ml Wasser gelöst, und der Lösung werden

nach Abkühlen auf 0 °C 13,2 g (0,742 Mol) Cyanamid portionenweise zugegeben. Der klaren und farblosen Cyanamidlösung werden 115,0 g (0,742 Mol) δ-Chlorvaleriansäurechlorid während einer Stunde bei 0 bis 8 °C zugetropft, wobei eine starke Exothermie festgestellt wird und deshalb intensiv gekühlt werden muss. Nach beendetem Zutropfen wird während einer weiteren Stunde bei 0 °C gerührt. Anschliessend werden 740 ml Methylenchlorid zugegeben, und das Reaktionsgemisch wird während einer Stunde auf Rückflusstemperatur (40 °C) erwärmt. Die beiden Phasen werden im Scheidetrichter getrennt. Die wässerige Phase wird nochmals mit 740 ml Methylenchlorid extrahiert.

Die vereinigten organischen Phasen werden mit Natriumsulfat getrocknet, filtriert und das Lösungsmittel am Wasserstrahlvakuum entfernt.

Ausbeute: 79,6 g (86,4 % d.Th.). Das Rohprodukt ist eine schwach gelbliche Flüssigkeit, welche laut Gaschromatogramm 79,95 % N-Cyanpiperidon enthielt. Ein Isomeres des N-Cyanpiperidons wurde nicht nachgewiesen. Nach Hochvakuumdestillation werden 44,0 g (47,8 % d.Th.) N-Cyanpiperidon, das laut Gaschromatogramm einen Gehalt von 99,19 % an N-Cyanpiperidon aufweist, als schwach gelbliche klare Flüssigkeit vom Siedepunkt 125 °C/13,3 Pa erhalten.

**Patentansprüche**

1. Verfahren zur Herstellung von N-Cyanlactamen der Formel

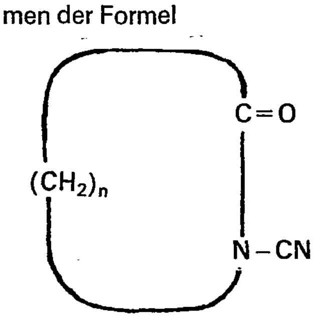

(I),

worin n eine ganze Zahl von 3 bis 5 bedeutet und die Methylenkohlenstoffatome gegebenenfalls durch eine oder zwei Methyl- oder Äthylgruppen substituiert sind oder zwei benachbarte Methylenkohlenstoffatome Bestandteil eines $C_5$–$C_8$ cycloaliphatischen Ringes oder Benzolringes sind, dadurch gekennzeichnet, dass man eine Verbindung der Formel II

$$X-\overset{\overset{\displaystyle O}{\|}}{C}-(CH_2)_n-X \qquad (II),$$

worin n eine ganze Zahl von 3 bis 5 ist, die Methylenkohlenstoffatome gegebenenfalls durch eine oder zwei Methyl- oder Äthylgruppen substituiert sind oder zwei benachbarte Methylenkohlenstoffatome Bestandteil eines $C_5$–$C_8$ cycloaliphatischen Ringes oder Benzolringes sind, und die Symbole X unabhängig voneinander für ein Halogenatom stehen, in Gegenwart eines organischen Halogenwasserstoffakzeptors mit Cyanamid oder einem Cyanamidsalz umsetzt.

2. Verfahren nach Anspruch 1, dadurch gekennzeichnet, dass man Verbindungen der Formel II umsetzt, welche keine Substituenten oder Ringe aufweist.

3. Verfahren nach Anspruch 1, dadurch gekennzeichnet, dass man 1 Mol der Verbindung der Formel II mit 1 Mol Cyanamid in Gegenwart von 2 Mol eines anorganischen Halogenwasserstoffakzeptors oder 1 Mol eines Cyanamidsalzes in Gegenwart von 1 Mol eines anorganischen Halogenwasserstoffakzeptors umsetzt.

4. Verfahren nach Anspruch 1, dadurch gekennzeichnet, dass man Verbindungen der Formel II umsetzt, in welcher die Symbole X unabhängig voneinander Chlor oder Brom bedeuten.

5. Verfahren nach Anspruch 1, dadurch gekennzeichnet, dass man Natriumhydroxid oder Kaliumhydroxid als Halogenwasserstoffakzeptor verwendet.

6. Verfahren nach Anspruch 1, dadurch gekennzeichnet, dass man äquivalente Mengen der Reaktanten bei 0–10 °C zusammenführt und das Reaktionsgemisch anschliessend auf 30–100 °C erwärmt.

7. Verfahren nach Anspruch 1, dadurch gekennzeichnet, dass die Umsetzung in einer wässrigen Lösung erfolgt.

**Revendications**

1. Procédé de préparation de N-cyanolactames répondant à la formule I:

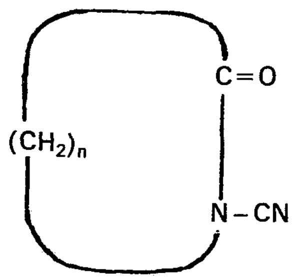

(I),

dans laquelle n représente un mombre entier de 3 à 5 et les atomes de carbone méthyléniques portent éventuellement un ou deux radicaux méthyles ou éthyles ou deux atomes de carbone méthyléniques voisins font partie d'un noyau cycloaliphatique en $C_5$–$C_8$ ou d'un noyau benzénique, procédé caractérisé en ce qu'on fait réagir un composé répondant à la formule II:

$$X-\overset{\overset{\displaystyle O}{\|}}{C}-(CH_2)_n-X \qquad (II).$$

dans laquelle n représente un nombre de 3 à 5, les atomes de carbone méthyléniques portent éventuellement un ou deux radicaux méthyles ou éthyles, ou deux atomes de carbone méthyléniques voisins font partie d'un noyau cycloaliphatique en $C_5$–$C_8$ ou d'un noyau benzénique, et les symboles X représentent chacun, indépendamment l'un de l'autre, un atome d'halogène, en présence d'un accepteur minéral d'halogénure d'hydrogène, avec le cyanamide ou un sel du cyanamide.

2. Procédé selon la revendication 1, caractérisé

en ce qu'on fait réagir des composés de formule II qui ne contiennent ni substituants ni noyaux.

3. Procédé selon la revendication 1, caractérisé en ce qu'on fait réagir 1 mole d'un composé de formule II avec 1 mole de cyanamide en présence de deux moles d'un accepteur minéral d'halogénure d'hydrogène ou avec 1 mole d'un sel du cyanamide en présence de 1 mole d'un accepteur minéral d'halogénure d'hydrogène.

4. Procédé selon la revendication 1, caractérisé en ce qu'on fait réagir des composés de formule II dans lesquels les symboles X représentent chacun, indépendamment l'un de l'autre, le chlore ou le brome.

5. Procédé selon la revendication 1, caractérisé en ce qu'on utilise l'hydroxyde de sodium ou l'hydroxyde de potassium comme accepteur d'halogénure d'hydrogène.

6. Procédé selon la revendication 1, caractérisé en ce qu'on met en présence des quantités équivalentes des partenaires réactionnels, à une température de 0 à 10 °C, puis on chauffe le mélange réactionnel à 30–100 °C.

7. Procédé selon la revendication 1, caractérisé en ce qu'on effectue la réaction en solution aqueuse.

## Claims

1. A process for the production of a N-cyanolactam of the formula

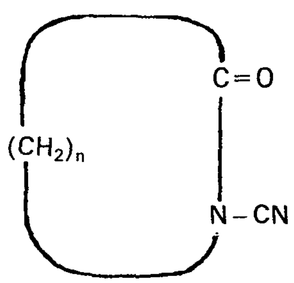

(I),

wherein n is an integer from 3 to 5 and the methylene carbon atoms are unsubstituted or substituted by one or two methyl or ethyl groups, or two adjacent methylene carbon atoms form part of a $C_5$–$C_8$ cycloaliphatic ring or a benzene ring, which process comprises reacting a compound of the formula II

$$X-\overset{\overset{\large O}{||}}{C}-(CH_2)_n-X \qquad (II),$$

wherein n is an integer from 3 to 5, the methylene carbon atoms are unsubstituted or substituted by one or two methyl or ethyl groups, or two adjacent methylene carbon atoms form part of a $C_5$–$C_8$ cycloaliphatic ring or a benzene ring, and each of the symbols X independently is a halogen atom, in the presence of an inorganic hydrogen halide acceptor, with cyanamide or a salt thereof.

2. A process according to claim 1, wherein there is used a compound of the formula II which contains no substituents or rings.

3. A process according to claim 1, wherein 1 mole of the compound of formula II is reacted with 1 mole of cyanamide, in the presence of 2 moles of an inorganic hydrogen halide acceptor, or with 1 mole of a cyanamide salt, in the presence of 1 mole of an inorganic hydrogen halide acceptor.

4. A process according to claim 1, wherein there is used a compound of the formula II, in which each of the symbols X independently is chlorine or bromine.

5. A process according to claim 1, wherein sodium hydroxide or potassium hydroxide is used as hydrogen halide acceptor.

6. A process according to claim 1, wherein equivalent amounts of the reactants are added to each other at 0°–10 °C and the reaction mixture is subsequently heated to 30°–100 °C.

7. A process according to claim 1, wherein the reaction is carried out in an aqueous solution.